# EUROPEAN PATENT SPECIFICATION

(11) **EP 1 573 001 B1**
(45) Date of publication and mention of the grant of the patent: **04.04.2007**
(21) Application number: 03812529.0
(22) Date of filing: 04.12.2003
(51) Int. Cl.: C12N 11/04, C12N 11/16

(54) **YEAST TREATMENT**
HEFEBEHANDLUNG
TRAITEMENT DE LEVURES

(30) Priority: 12.12.2002 AU 2002953285
(43) Date of publication of application: 14.09.2005
(73) Proprietor: Protech Research PTY LTD, New South Wales 2092 (AU)
(72) Inventor: PATANE, Michael, Seaforth, New South Wales 2092 (AU)
(74) Representative: Winckels, Johannes Hubertus F.
(86) International application number: PCT/AU2003/001619
(87) International publication number: WO 2004/053118

(56) References cited:
- WO-A-85/04880
- WO-A-95/31183
- WO-A-98/58630
- ALLSHIRE R C: "Introduction of large linear minichromosomes into Schizosaccharomyces pombe by an improved transformation procedure" PROCEEDINGS OF THE NATIONAL ACADEMY OF SCIENCES OF THE UNITED STATES OF AMERICA, vol. 87, no. 11, 1990, pages 4043-4047, XP002345219 ISSN: 0027-8424
- SPARRER H E ET AL: "Evidence for the prion hypothesis: Induction of the yeast (PSI+) factor by in vitro-converted Sup35 protein" SCIENCE (WASHINGTON D C), vol. 289, no. 5479, 28 July 2000 (2000-07-28), pages 595-599, XP002345220 ISSN: 0036-8075
- DOUMA A C ET AL: "Liposome-mediated introduction of proteins into protoplasts of the yeast Hansenula polymorpha as a possible tool to study peroxisome biogenesis" YEAST, vol. 6, no. 2, 1990, pages 99-106, XP002345221 ISSN: 0749-503X
- KIM J-H ET AL: "Production of ribonucleotides by autolysis of Hansenula anomala grown on Korean ginseng steaming effluent" JOURNAL OF BIOSCIENCE AND BIOENGINEERING, vol. 93, no. 3, 2002, pages 318-321, XP002345222 ISSN: 1389-1723
- LI, W. M. ET AL: 'Intermembrane transfer of polyethylene glycol-modified phosphatidylethanolamine as a means to reveal surface-associated binding ligands on liposomes' BIOCHEMICA ET BIOPHYSICA ACTA vol. 1513, 2001, pages 193 - 206, XP004255711
- PERRIE, Y. ET AL: 'Liposome-mediated DNA vaccination, the effect of vesicle composition' VACCINE vol. 19, 2001, pages 3301 - 3310, XP004234205
- MILLER, C. R. ET AL: 'Liposome-Cell Interaction in Vitro: Effect of liposome Surface Charge on the Binding and Endocytosis of Conventional and sterically Stabilized Liposomes' BIOCHEMISTRY vol. 37, 1998, pages 12875 - 12883, XP002924600
- ANZAI, K ET AL: 'Frequent Fusion of Liposomes to a Positively Charged Planar Bilayer without Calcium Ions' J.BIOCHEM vol. 114, 1993, pages 487 - 491, XP009054000
- ATAMATATOS, L ET AL: 'Interactions of catinic lipid Vesicles with Negatively Charged Phospholipid Vesicles and biological membranes' BIOCHEMISTRY vol. 27, 1988, pages 3917 - 3925, XP000601444

## Description

### Field of the invention

The invention relates to liposomes for introducing a molecule into a yeast cell and to processes for making yeast cells comprising an exogenous molecule.

### Background of the invention

Yeast species such as those in the genera Kluyveromyces and Saccharomyces have a number of uses in the food and beverage industries such as, for example, for providing fermentation products and for providing flavouring enhancers.

There is a demand for new and improved strains of yeasts in these industries and presently, such strains are provided either by culturing and selecting a strain according to a particular phenotype, or by using recombinant DNA technology to introduce one or more genes into a cell to provide the particular phenotype. Although such processes are useful for generating yeast strains, certain limitations apply to the suitability of these processes for this purpose. For example, culturing and selecting a strain according to a particular phenotype is time consuming, expensive and does not allow one to create a desired phenotype. Recombinant DNA technology allows one to create a phenotype, however yeast cells produced by these technologies are typically recognised as genetically modified organisms, and this often has deleterious consequences for products produced from such organisms.

Often, the critical factor for providing the desired effector function, and accordingly, the useful phenotype in a new yeast strain, is the action of one or more macromolecules such as proteins. Accordingly, it would be advantageous if one were able to transfect, or in other words, to introduce macromolecules such as proteins into a yeast cell to provide the desired phenotype, as this would avoid the limitations of culture and selection techniques, and also the techniques based on recombinant DNA technology..

One way of transfecting macromolecules such as proteins into cells is to use liposomes. A liposome is a structure comprising one or more concentric spheres of a lipid bilayer that enclose an aqueous compartment. To date, it has not been possible to generate a liposome that is capable of transfecting a macromolecule, such as an active enzyme, into a yeast cell.

Flavouring agents or enhancers, specifically, ribonucleotides and monosodium glutamate, are typically produced from yeast cells according to the following process. First, yeast cells are lysed to release one or more fractions containing RNA. Second, the fractions containing RNA are contacted with 5'-phosphodiesterase to produce ribonucleotides. Finally, the fractions are heated to prevent further degradation of the ribonucleotides. These processes are often expensive, are time consuming and requiring technical expertise, and are notorious for producing a product with low market value.

Further, ribonucleotides are conventionally used as a flavouring enhancer by adding the ribonucleotides as an ingredient to a mixture for preparing a food or foodstuff. One disadvantage of this is that in some circumstances, the ribonucleotide must be disclosed on a label for a food product as an additive. This has consequences for the market value of the food product.

In view of the above, there is a need for improvements in yeast cells, including yeast cells for providing flavouring enhancers.

### Summary of the invention

The invention seeks to at least minimise one or more of the above identified problems or limitations and/or to provide improvements in yeast cells, especially yeast cells for use in providing flavouring enhancers.

In one aspect, the invention provides a liposome for fusing with a yeast cell. The liposome is characterised in that 40-50 molar % of the liposome lipid bilayer is phosphatidyl choline (PC), 10-20 molar % of the liposome lipid bilayer is a cationic amphiphile, about 10 molar % of the liposome lipid bilayer is a sterol and about 30 molar % of the liposome lipid bilayer is phosphatidyl ethanolamine (PE) and/or dioleoylphosphatidylethanolamine (DOPE).

A process for preparing a yeast cell for fusion with a liposome is described herein. The process comprises treating the yeast cell to form a yeast cell spheroplast or protoplast.

Described herein is a process for introducing an exogenous molecule into a yeast cell. The process comprises contacting a yeast cell spheroplast or protoplast with a liposome comprising the exogenous molecule in conditions for permitting the spheroplast or protoplast to receive the liposome. The liposome for use in the process is as described above.

In another aspect, the invention provides a process for producing a yeast cell comprising an exogenous molecule. The process comprises contacting a yeast cell spheroplast or protoplast with a liposome comprising the exogenous molecule in conditions for permitting the spheroplast or protoplast to receive the liposome. The liposome for use in the process is as described above.

Described herein is a yeast cell produced by the above described process as well as a use of a yeast cell as described above for producing a food, foodstuff, additive, or an ingredient for producing a food or foodstuff.

In another aspect, the invention provides a liposome capable of fusing with a yeast cell. The liposome is characterised in that 40-50 molar % of the liposome lipid bilayer is PC, 10-20 molar % of the liposome lipid bilayer is 1,2-dioleoyl-*sn-*glycero-3-trimethylammonium-propane (DOTAP), about 10 molar % of the liposome lipid bilayer is ergosterol and about 30 molar % of the liposome lipid bilayer is PE and/or DOPE. The liposome further comprises a 5' phosphodiesterase for hydrolysing a RNA molecule to form a ribonucleotide in a yeast cell.

In another aspect, the invention provides a liposome capable of fusing with a yeast cell. The liposome is characterised in that 40-50 molar % of the liposome lipid bilayer is PC, 10-20 molar % of the liposome lipid bilayer is DOTAP, about 10 molar % of the liposome lipid bilayer is ergosterol and about 30 molar % of the liposome lipid bilayer is PE and/or DOPE. The liposome further comprises 5'-adenyl deaminase for deamination of adenylate to form a 5'-inosine phosphate nucleotide.

Described herein is a process for producing a liposome comprising an enzyme, as described above. The process comprises forming a dispersion of PC, DOPE and/or PE, ergosterol and DOTAP in an organic solvent, drying the dispersion to form dried lipids and redispersing the dried lipids in an aqueous medium comprising the enzyme to form a liposome.

In another aspect, the invention provides a process for producing a yeast cell comprising a 5' phosphodiesterase for hydrolysing a RNA molecule to form a ribonucleotide. The process comprises contacting a yeast cell spheroplast or protoplast with a liposome comprising an enzyme as described above, to permit the spheroplast or protoplast to receive the liposome.

Described herein is a yeast cell comprising a 5' phosphodiesterase, for hydrolysing a RNA molecule to form a ribonucleotide. The yeast cell is one produced by the process described above.

In another aspect, the invention provides a process for producing a yeast cell comprising 5'-adenyl deaminase for deamination of adenylate to form a 5'-inosine phosphate nucleotide. The process comprises contacting a yeast cell spheroplast or protoplast with a liposome comprising an enzyme as described above, to permit the spheroplast or protoplast to receive the liposome.

Described herein is a yeast cell comprising 5'-adenyl deaminase for deamination of adenylate to form a 5'-inosine phosphate nucleotide.

A process for producing a flavour enhancer in a yeast cell is described herein. The process comprises treating a yeast cell to form a spheroplast or protoplast, contacting the spheroplast or protoplast with a liposome comprising an enzyme for hydrolysis of an RNA molecule to form a ribonucleotide, in conditions for permitting the spheroplast or protoplast to receive the liposome and providing conditions for permitting hydrolysis of an RNA molecule in a yeast cell by the enzyme of the liposome, to produce the flavour enhancer.

A process for producing a flavour enhancer in a yeast cell is described herein. The process comprises treating a yeast cell to form a spheroplast or protoplast, contacting the spheroplast or protoplast with a liposome comprising an enzyme for deamination of adenylate to form a 5'-inosine phosphate nucleotide, in conditions for permitting the spheroplast or protoplast to receive the liposome and providing conditions for permitting deamination of an adenylate in a yeast cell by the enzyme of the liposome, to produce the flavour enhancer.

### Detailed description of the embodiments

As described herein, the inventor has produced a liposome that is capable of fusing with a yeast cell and, importantly, capable of transferring a molecule contained by the liposome to a yeast cell cytoplasm to permit the molecule to perform an intended function in the yeast cell. Accordingly, the invention provides a liposome characterised in that:
- 40-50 molar % of the liposome lipid bilayer is PC;
- 10-20 molar % of the liposome lipid bilayer is cationic amphiphile;
- about 10 molar % of the liposome lipid bilayer is sterol; and
- about 30 molar % of the liposome lipid bilayer is PE and/or DOPE.

In one embodiment, 50 molar % of the liposome lipid bilayer is PC. In another embodiment, 20 molar % of the liposome lipid bilayer is cationic amphiphile. In another embodiment, about 30 molar % of the liposome lipid bilayer is DOPE. In another embodiment, about 30 molar % of the liposome lipid bilayer is PE.

Typically, the sterol is ergosterol, or a molecule with a similar structure for example, cholesterol.

Steryl amine or DOTAP are particularly useful in the liposome of the invention as a cationic amphiphile.

The liposome lipid bilayer may further comprise oleic acid. In one embodiment, about 5 molar % of the liposome lipid bilayer is oleic acid. Typically, where the liposome lipid bilayer further comprises oleic acid, less than 30 molar % of the liposome lipid bilayer is PE and/or DOPE.

The following liposomes are particularly useful for fusion with, and transfer of a macromolecule contained by the liposome to a yeast cell:
(a) a liposome characterised in that:
   - 50 molar % of the liposome lipid bilayer is PC;
   - about 30 molar % of the liposome lipid bilayer is DOPE;
   - 10 molar % of the liposome lipid bilayer is DOTAP; and
   - about 10 molar % of the liposome lipid bilayer is ergosterol.
(b) a liposome characterised in that:
   - 40 molar % of the liposome lipid bilayer is PC;
   - about 30 molar % of the liposome lipid bilayer is DOPE;
   - 20 molar % of the liposome lipid bilayer is DOTAP; and
   - about 10 molar % of the liposome lipid bilayer is ergosterol.
(c) a liposome characterised in that:
   - 50 molar % of the liposome lipid bilayer is PC;
   - about 30 molar % of the liposome lipid bilayer is PE
   - 10 molar % of the liposome lipid bilayer is DOTAP; and
   - about 10 molar % of the liposome lipid bilayer is ergosterol.
(d) a liposome characterised in that:
   - 40 molar % of the liposome lipid bilayer is PC;
   - about 30 molar % of the liposome lipid bilayer is PE;
   - 20 molar % of the liposome lipid bilayer is DOTAP; and
   - about 10 molar % of the liposome lipid bilayer is ergosterol.

Typically, the liposome of the invention further comprises a molecule to be introduced into a yeast cell. The molecule is contained by the liposome, in the sense that it may be encapsulated by the liposome; i.e. it may be confined within an aqueous compartment that is defined by the liposome lipid bilayer. Alternatively, the molecule may be embedded in the lipid bilayer and/or may protrude into the aqueous compartment defined by the bilayer and/or protrude from the surface of the liposome lipid bilayer for contact with the yeast cell.

The molecule further comprised by the liposome is an "exogenous molecule", in the sense that it is one that has not been produced by, or derived or obtained from the yeast cell to which the molecule is to be transferred. Accordingly, synthetically or artificially produced molecules, including those not found in nature, and molecules produced by cells other than yeast cells are exogenous molecules.

It will be understood however that an exogenous molecule may be identical to a molecule that is produced by a yeast cell. For example, a 5' phosphodiesterase that is obtained from a yeast cell is an exogenous molecule to a yeast cell to which the enzyme is to be transferred in the circumstance that the enzyme was not produced by or obtained from that yeast cell.

It will be understood that the molecule further comprised in the liposome can be any molecule that is capable of being contained by a liposome and that would impart a desirable effect to a yeast cell. Examples of such molecules are those useful for producing fermentation products such as proteins, particularly, enzymes, amino acids, nucleic acids, sugars, organic compounds and mono sodium glutamate.

Further, it will be understood that in particular circumstances, the above described liposome may not comprise a molecule to be introduced to a yeast cell. More specifically, in some circumstances, it is recognised that the liposome has particular utility as a surface for reaction of one or more substrates. In such circumstances, the liposome of the invention is not provided with a further molecule to be introduced into a yeast cell.

As described herein, it is believed that the liposome of the invention transfers, or in other words, introduces a molecule contained by the liposome to the yeast cell by endocytosis. Accordingly, typically the liposome has a diameter for permitting endocytosis of the liposome by a yeast cell. A suitable diameter for the liposome is between 100 and 400 nm and a particularly useful liposome is one having a diameter between 200 and 400 nm.

It is believed that destabilisation of the liposome lipid bilayer is an important step in the process by which the liposome fuses with an endosomal bilayer of the yeast cell, leading to transfer of a molecule that may be contained by a liposome to the yeast cell. One approach for causing destabilisation of the liposome lipid bilayer is to adapt the liposome so that the lipid bilayer is sensitive to fluctuations in pH. Typically, the liposome of the invention is adapted for destabilisation of the liposome lipid bilayers at the pH of a yeast endosome. A liposome comprising lipid bilayers adapted for destabilisation at a pH of about 5.0 to 6.0 is particularly useful.

In experiments leading to the invention, the inventor recognised that certain manipulations of a yeast cell may be particularly useful for enabling fusion of the liposome with the yeast cell, for example, for transfer of a molecule contained by the liposome to the yeast cell. More particularly, the inventor recognised that certain molecules, including α (1-6) and (1-3) mannan (yeast gum)/ protein complexes and β (1-6) and (1-3) glucan (yeast cellulose)/protein complex with a chitin backbone would need to be removed at least in part from the yeast cell, to form a yeast cell spheroplast, or removed more substantially, to form a yeast cell protoplast. As described herein, the outer lipid membrane of a yeast cell protoplast, otherwise known as the plasmalemma or plasma membrane contains primarily proteins, sterols, sphingolipids and phospholipids. The plasmalemma is sufficient for providing a barrier for regulation of transport of molecular species into and out of the yeast cell. Surprisingly, the inventor has found that the treatment described herein is sufficient for at least partial removal of yeast gum and yeast cellulose and yet is sufficiently sensitive to provide viable yeast cell spheroplasts or protoplasts that are capable of endocytosis of the liposome of the invention.

Described herein is a process for preparing a yeast cell for fusion with a liposome comprising the step of treating the yeast cell to form a yeast cell spheroplast, or a yeast cell protoplast.

Typically, the yeast cell is treated to form a yeast cell spheroplast or protoplast by contacting the yeast cell with one or more enzymes for at least partial digestion of yeast gum and yeast cellulose. Lyticase and β-glucuronidase are particularly suitable enzymes for this purpose. The process may comprise the further step of isolating a spheroplast or protoplast. A density gradient is particularly useful for this purpose. Typically, the yeast cells for treatment in the process to produce the yeast cell spheroplasts or protoplasts are cells existing in the exponential growth phase. Before treatment of the yeast cells to form a yeast cell spheroplast or protoplast, the yeast cells may be washed to remove culture media from the yeast cells.

Described herein is a process for introducing an exogenous molecule into a yeast cell spheroplast or protoplast. The process comprises contacting a yeast cell spheroplast or protoplast with a liposome comprising the molecule in conditions for permitting the spheroplast or protoplast to receive the liposome. Typically the liposome for use in the process is as described above.

As described herein, the inventor has observed that the liposome of the invention is received by a yeast cell spheroplast or protoplast by endocytosis. Typically, the yeast cell spheroplast or protoplast and liposome are contacted to permit the spheroplast or protoplast to receive the liposome, or in other words, to endocytose the liposome, at about 37°C. As described herein, the spheroplast or protoplast and liposome may be contacted for endocytosis in a buffer having an osmolarity compatible for a yeast cell.

The invention provides a process for producing a yeast cell spheroplast or protoplast comprising an exogenous molecule. The process comprises contacting a yeast cell spheroplast or protoplast with a liposome comprising the molecule to permit the spheroplast or protoplast to receive the liposome. The liposome for use in the process is as described above.

The yeast cell spheroplast or protoplast and liposome may be contacted for permitting the spheroplast or protoplast to receive the liposome, according to the conditions described above. The process may comprise the further step of culturing a yeast cell spheroplast or protoplast comprising the exogenous molecule to provide a yeast cell.

Described herein is a yeast cell, spheroplast or protoplast produced by the above described process. Particularly suitable cells, spheroplasts and protoplasts for use in the above described processes are of genera Kluyveriomyces and Saccharomyces.

Described herein is a use of a yeast cell as described above for producing a food, foodstuff, additive or an ingredient for producing a food or foodstuff. The use of the yeast cell may be a process comprising the step of contacting the yeast cell with a composition to produce a food, food stuff, additive or an ingredient for producing a food or food stuff.

Also described herein is a food, food stuff, additive, or ingredient for production of a food or food stuff, produced by a yeast cell as described above. Typically, the food, food stuff, additive or ingredient for production of a food or food stuff, is a product produced by fermentation or Malliard reaction. Examples include products that contain hydrolysed vegetable protein.

A particularly useful liposome of the invention comprises 5' phosphodiesterase for hydrolysing RNA molecules in a yeast cell to produce 5'-ribonucleotides. Accordingly, the invention provides a liposome characterised in that:
- 40-50 molar % of the liposome lipid bilayer is PC,
- 10-20 molar % of the liposome lipid bilayer is DOTAP,
- about 10 molar % of the liposome lipid bilayer is ergosterol and
- about 30 molar % of the liposome lipid bilayer is PE and/or DOPE; wherein the liposome comprises 5'' phosphodiesterase.

This liposome is particularly useful, because as described further herein, such a liposome can transfect a 5'-phosphodiesterase into a yeast cell to permit the 5'-phosphodiesterase to hydrolyse RNA molecules in the cytoplasm to produce ribonucleotides. Accordingly, a particular advantage of such a liposome is that ribonucleotides can be provided for use as flavouring enhancers without the preliminary steps of purifying RNA from a yeast cell lysate, digesting the RNA with the enzyme, and isolating the ribonucleotide products. Other enzymes or compounds capable of forming a compound for providing flavouring to a food or foodstuff, or for providing flavouring to an ingredient for preparing a food or foodstuff may be comprised in the liposome. An example of such an enzyme is 5'-adenyl deaminase.

A further particularly useful liposome of the invention comprises 5'-adenyl deaminase for deamination of adenylate to produce a 5'-inosine phosphate nucleotide. Accordingly, the invention provides a liposome characterised in that:
- 40-50 molar % of the liposome lipid bilayer is PC,
- 10-20 molar % of the liposome lipid bilayer is DOTAP,
- about 10 molar % of the liposome lipid bilayer is ergosterol and
- about 30 molar % of the liposome lipid bilayer is PE and/or DOPE; wherein the liposome comprises 5'-adenyl deaminase.

A process for producing a liposome comprising an enzyme, as described above is described herein. The process comprises forming a dispersion of PC, DOPE and/or PE, ergosterol and DOTAP in an organic solvent, drying the dispersion to form dried lipids and redispersing the dried lipids in an aqueous medium comprising the enzyme to form a liposome. As described herein, a 2 : 1 chloroform-methanol solution is particularly useful as an organic solvent for forming a dispersion of the lipids. Typically, the process comprises a further step of isolating liposomes having a diameter of about 400 nanometres.

The invention also provides a process for producing a yeast cell spheroplast or protoplast comprising an enzyme as described above. The process comprises contacting a yeast cell protoplast with a liposome comprising the enzyme, to permit the spheroplast or protoplast to receive the liposome. As described herein, the inventor has observed that the liposome of the invention is received by a yeast cell spheroplast or protoplast by endocytosis. Typically, the yeast cell spheroplast or protoplast and liposome are contacted to permit the spheroplast or protoplast to receive the liposome, or in other words, to endocytose the liposome, at about 37°C. As described herein, the spheroplast or protoplast and liposome are contacted for endocytosis in a buffer having an osmolarity that is compatible with a yeast cell.

Described herein is a yeast cell comprising an enzyme for hydrolysing a RNA molecule to form a ribonucleotide. The yeast cell is one produced by the process described above.

Also described herein is a yeast cell comprising an enzyme for deamination of adenylate to form 5'-inosine phosphate nucleotide. The yeast cell is one produced by the process described above.

Described herein is a process for producing a flavouring enhancer in a yeast cell. The process comprises treating a yeast cell to form a spheroplast or protoplast, contacting the spheroplast or protoplast with a liposome comprising an enzyme as described above, to permit the spheroplast or protoplast to receive the liposome and providing conditions for function of the enzyme in the yeast cell by the enzyme of the liposome, to produce the flavouring enhancer.

Also described herein is a flavouring enhancer produced by the above described process. The flavouring enhancer produced by the process of the invention is typically a ribonucleotide such as 5'-guanosine monophosphate, 5'-cytosine monophosphate, 5'-adenosine monophosphate or 5'-uracil monophosphate. Preferably the ribonucleotide is 5'-guanosine monophosphate or 5'-inosine monophosphate. The flavouring enhancer may also be a compound such as monosodium glutamate.

Described herein is a food or food stuff, or ingredient for producing a food or food stuff, comprising a flavouring agent or enhancer produced by the above described process. The food or food stuff, or ingredient, or additive for producing a food or food stuff produced by the process described herein is typically a leavened product or savoury product or beverage produced by fermentation or Malliard reaction, or containing a product from either of these reactions.

Described herein is a composition comprising a liposome as described above.

### Examples

### Example 1: Materials & Equipment

Soy phosphatidylcholine (PC), dioleoylphosphatidylethanolamine (DOPE), soy phosphatidylethanolamine (PE), 1,2-dioleoyl-*sn*-glycero-3-trimethylammonium-propane (DOTAP), ergosterol (ERGO), calcein, fluorescein isothiocyanate dextran, (FITC-FD 250S), dextrose, trisma base, ficoll-type 70, fluorinert FC-77, melting point bath oil, I-sucrose-ul-14C, silicone oil AR 200, lyticase and β-glucuronidase - type H2 were all obtained from Sigma Aldrich (Castle Hill, Australia), Sepharose CL-4B was obtained from Amersham Biosciences (Castle Hill, NSW). Sorbitol was purchased from Med-Chem (Kew, Vic). Yeast extract and bacterial peptone were purchased from Oxoid Chemicals (Heidelberg, Vic). Saccharomyces cerevisiae (YNN 281) and K. marxianus (FRR 1337) were kindly provided by Food Science Australia (North Ryde NSW). All remaining chemicals and solvents were of HPLC grade.

A Buchi rotary evaporator and Heto FD-3 freeze drier were used to initially dry and then remove all traces of organic solvents from the prepared phospholipid films with re hydration occurring in a Braun Certomat WT temperature controlled shaking water bath. Formation of multilamellar vesicles (MLV) was initially undertaken in a Braun 1200 bath sonicator with the addition of 2mm glass beads to aid in the removal of the dried lipid from the walls of the flask. Size reduction of the liposomes from MLVs to large unilamellar vesicles (LUV) was undertaken in an Avestin Lipo-fast basic with stabiliser extruder incorporating 400nm polycarbonate membrane filters after a series of cyclic freezing and thawing steps in the range of -80°C to 40°C utilising a laboratory freezer and a heated water bath. This step aimed to increase the capture volume with the formed liposome. The LUVs were separated from any unencapsulated material by gel filtration using an Amersham Pharmacia AKAT gradient processing FPLC system complete with a 900-model monitor, lamp and detector (set at 280nm), 920 model pump and incorporating a Frac 950 fraction collector interfaced to a Compaq Desk Pro Pentium III computer supporting Unicorn analytical software. The column used for the chromatograph was a K9-30 column packed with Sepharose CL 4B beads (45um to 165um) and fitted with two 25um supporting filters. The column was packed using a RK16/26 packing reservoir and filled using a variable speed peristaltic pump. The particle size of the formed LUV liposomes was estimated on a Malvern Mastersizer-X-long bed particle analyser interfaced to an ACO Pentium II computer supporting Mastersizer Version 2.18 analytical software. A Bioline orbital shaker was used to incubate the yeast with washing, pelletising and density separations for cells and protoplasts undertaken on a Beckman J2-H2 centrifuge utilising a fixed head JA 20 rotor as well as a JS-13 swing bucket head rotor specifically for the separation of protoplast. Confirmation of protoplast viability was undertaken by C 14- sucrose uptake measured on a Packard 1600TR liquid scintillation analyser after pelletising the protoplasts on a Beckman 152 microfuge through a silicon oil gradient.

Confirmation of fluorescent endocytosis was undertaken on a Leica TCS-4D Confocal Microscope fitted with a Krypton-Argon mixed gas laser set at an excitation wavelength of 488nm which was interfaced to a Dell Pentium 2 computer supporting Leica Scan-ware imaging software. Detailed cell imaging to identify the position of liposomes within the cytoplasm and plasma membrane was undertaken on a Philips CM 100 Transmission Electron Microscope with images captured on a Gatan Dual Vision Digital camera.

### Example 2: Creation of Liposomes

### Glassware:

Prior to use in the trials all glassware was washed in a phosphate free detergent followed by immersion in 5M nitric acid and rinsed in Milli Q water to remove any trace of residual proteinaceous material, lipid or salt in an effort to avoid unwanted oxidation of the phospholipids or the possible insertion of any extraneous materials into the formed lipid bilayers which would change the charge or packing density of the created lipsomes.

### Preparation of standard phospholipid solutions:

Stock solutions of 20mg/ml were prepared for PC, PE, DOPE, DOTAP and ERGO. They were prepared by dissolving 20mg of each lipid in to 1ml of a 2:1 (v/v) solution of chloroform and methanol which had been filtered through a 0.45um Millipore filter and stored at 4°C until use.

Only glass flasks, beakers, syringes and stainless steel needles were used in the preparation of these mixtures to avoid the possible migration of plastic monomers from the dispensing pipette tips or filters which could contaminate and alter the packing configuration of the phospholipids micelles which could result in premature leakage of the entrapped materials upon storage of the liposome suspension.

### Preparation of the dried lipid film:

To a washed 100 ml Quick fit round bottom flask was added 5ml of the 2:1 chloroform-methanol solution to ensure adequate dispersion of the phospholipids. A total of 250µl of mixed lipids from the 20mg/ml stock solutions was then added and gently mixed for 1 hour. The flasks were wrapped in aluminium foil to protect the lipids against oxidation by UV light and the headspace was nitrogen flushed for 5 minutes to remove oxygen from the headspace prior to attaching a flask to the Buchi rotary evaporator. The solvents were evaporated under vacuum for 90 minutes at 60rpm in a water bath set at 37°C, which is above the phase transition temperature (Tm°C) for the phosphatidylcholine component thereby ensuring all lipids were in the liquid crystalline phase allowing for a uniform dispersion. The evaporator was also covered in a black plastic film to further ensure a minimal passage of light. To ensure the removal of all solvents from the dried lipids, the flasks were finally freeze dried at -52°C at 0.001Mbar pressure for 2 hours then re-flushed with nitrogen, sealed and stored at -80°C until required for re hydration.

### Hydration of the dried lipid film and the encapsulation of a fluorescent dye:

The dried phospholipid films were hydrated in a two-stage process to avoid the precipitation or flocculation of the charged cationic lipid DOTAP which is sensitive to millimolar concentration of polyanions such as calcein, phosphate or EDTA as well as the presence of either monovalent or divalent cations in concentrations higher than five millimolar. The separation phenomenon of cationic lipids were observed during our work with the process investigated and corrected during our trials by hydration of the film initially in Milli Q water at 37°C for 30 minutes to initiate the formation of a micelle complex. This was then followed by a 30 minute to 1 hour hydration with the desired encapsulent in an appropriately buffered solution at pH 7.2 to induce a cationic sensitivity onto the liposome. Our initial trials used a 20mM solution of calcein in 10mM of Trisma adjusted to pH 7.2 and then continued with a 10mg/ml solution of fluorescein isothiocyanate dextran FD 250S to ensured the passive movement of a smaller molecule across the plasma membrane of our formed protoplasts was avoided. It was identified in earlier trials that when the smaller molecular weight calcein was incubated alone with the formed protoplasts there was some passive migration of this molecule across the plasma membrane or our cells producing a false positive result. Substitution of calcein with the FITC 250kDA dextran eliminated this situation and provided accurate data on liposomal induced endocytosis. The hydrated lipids were then cyclic frozen and thawed 3 times from -80°C to 37°C in an effort to increase the capture of volume of the fluorescent dye within the lipid bilayers while avoiding the break down of the FITC conjugated dextran. A brief bath sonication for 10 minutes was undertaken to finalise development of the MLVs while reducing any excessive aggregation between the micelles. Each solution was then extruded in an Avestin Lipo fast basic with stabiliser extruder.

### Extrusion and isolation of the Large Unilamellar Vesicles by Gel Filtration:

The Avestin lipofast basic extruder is a hand driven extrusion device with a capacity of 1 millilitre utilising 2 purpose built Hamilton gas tight syringes fitted to a pair of lour locked membrane supports that enclose a polycarbonate filter which is bound within a stainless steel housing . Prior to each use, the extruder components are washed in water than with a 2:1 chloroform: methanol solution to remove any residual lipid and rinsed again in Milli Q water and allowed to dry prior to assembly. Our experiment used a 400nm disposable polycarbonate filter that was fitted to the membrane supports with tongs to avoid contamination with ancillary lipids. Later work identified that 200nm filters may be more appropriate for an enhanced endocytotic entry of our liposomes across the cell wall based on transmission electron micrographs of large liposomes adhering to the outer membrane wall and smaller liposomes passing into the cytoplasm of the protoplasts. The drawback of this assumption is that the capture volume of the lipsome will also reduced. Upon assembly of the extruder, the syringes were individually filled with the 10mM Trisma buffer at pH 7.2 and extruded through the polycarbonate filter into the opposing syringe and the contents discarded. This was repeated with the other syringe but undertaken in the opposite direction with the intention of pre wetting the filer pad for an easier passage of the lipid solution through the membrane as well as removing any remaining dye or lipid which may have been bound within the casing of the extruder.

The extruder and the flasks containing the MLVs were immersed in a water bath at 37°C to preheat the apparatus and ensure the lipids were in a liquid crystalline state ensuring an easier passage through the extruder. The solutions were passed through the extruder in a forward and backward motion 23 times to achieve a uniform homogenous dispersion of LUVs. For each extrusion it was important to finish the procedure in the opposite syringe to avoid the re suspension of any trapped material which may be present on the filter during the first pass. The solutions, which became clearer on repeated extrusions, were filled into 2ml pre sterilised eppendorf tubes which were covered in aluminium foil and stored at 4°C prior to gel filtration.

The buffer for gel filtration consisted of a 1M sorbitol, 100mM potassium chloride, 25mM trisma base and 100uM magnesium chloride adjusted to ph 7.2. Prior to use, the buffer was filtered and degassed under vacuum for 10 minutes and prepared in Milli Q water. After filtration, the buffer was also boiled to remove any remaining air and held in a jacketed bath at 37°C to ensure the lipids were in a liquid phase during the filtration. The requirements for removing trapped air from the buffer prior to its use was to increasing the degree of separation between the liposomes and the free dye by avoiding the compression of the soft Sepharose packing beads. Prior to use, the column was equilibrated with three volumes of buffer to remove any residual ethanol or sodium azide which was used as a preservative within the FPLC lines and the Sepharose packed column, while not in use the columns were stored in the refrigerator at 4°C. The separation protocol used a flow rate of 0.5ml/min and two millilitres of liposome suspension were injected into the column and an elution efficiency of 95%. The process volume used was 79mls and the process time was 197 minutes.

### Example 3: Creation and confirmation of competent yeast protoplasts

### Preparation of culture media and incubation of yeast cells:

For the production of yeast protoplasts, a freeze-dried culture of Saccharomyces cerevisiae (YNN 281) was recussitated and then propagated for 20 hours at 30°C in a temperature controlled shaker set at 120rpm and grown in a sterilised YEPD media containing 1.5% (w/v) yeast extract, 2.0% (w/v) bacterial peptone and 2.0% (w/v) dextrose adjusted to pH 6.5. After incubation, the yeast cells were washed twice in an osmotically stabilised solution containing 0.65M potassium chloride, 25mM Trisma base and 100uM magnesium chloride adjusted to pH 6.5. The concentration requirement for this suspension protocol is based on an understanding of the intracellular osmotic pressure and concentration of resting yeast from the genus Saccharomyces cerevisiae as defined using freezing point depression data. Centrifugation of the harvested cells were undertaken at 4°C in a Beckman J2 H2 centrigue at 10,000rpm for 15 minutes.

It has been identified that the porosity and lipid content of the yeast plasma membrane can be altered for a facilitated endocytotic transport of higher molecular weight compounds if the conditions for time, temperature or pH are manipulated or the solutes in the supporting media were modified to enhance lipid membrane growth.

### The preparation of an enzyme solution and density gradients for the isolation of yeast protoplasts:

For the digestion of our yeast cell walls an enzyme digest was prepared containing 2 mg/ml of Lyticase and 1mg/ml of snail gut juice (β-Glucuronidase from Helix pomatia) in a solution of 0.65M potassium chloride, 25mM trisma base and 100uM magnesium chloride adjusted to pH 6.5. This is the same solution that was used for the washing and re suspension of the harvested yeast cells. The protoplasts were prepared from a 4 ml washed cell suspension with 1 ml of enzyme preparation added. Incubation was undertaken in a sealed conical flask and reacted for 3 hours at 37°C on a temperature controlled rotary shaker set at 120 rpm to produce our viable protoplasts.

A five-tier density gradient was then constructed in Falcon tubes to isolate protoplasts from intact cells and cell wall remnants in the range of 1.04g/l to 1.10g/l. Each solution comprised sorbital from 0.65M to 1M in concentration, 25mM of trisma base, 100uM of magnesium chloride and either 5 or 10% Ficoll in the densest fractions. Each solution was stabilised to pH 7.2 and a total of 5 ml of each solution was layered into the falcon tubes beginning with the densest fraction and finishing with the yeast - enzyme digest. The tubes were then capped and place in the Beckman centrifuge and spun for 15 minutes at 1000rpm and 4°C to isolate the protoplasts.

### Confirmation of yeast cell viability by C14 sucrose adsorption:

To ensure the competent nature of the formed protoplast to undertake endocytosis, the yeast cells were grown on a modified YEPD media in which the 2% (w/v) dextrose was replaced by 4% (w/v) sucrose as the carbon source to facilitate a mechanism for the absorption and transport of the sucrose C14 by the yeast cells. Protoplasts were formed as described above and isolated again utilising a sorbitol based density gradient. Silicon oil isolation of protoplasts is a convenient method for separating radio labelled cells from a sugar gradient solution. In this procedure cells are incubated in a radioactive solution for a specified period of up to 1 hour as seen in our trials and then spun through silicon oil at a specific density to separate them from scintillation counting. Seven micro tubes (400ul) were prepared for pelletising the protoplasts with each containing 10ul of Fluorinert FC 77 as a capture solution and 100ul of Silicon Oil AR 200 (Density of 1.05g/l) as the media to be used for the separation of sucrose C14 protoplasts. In the experiment 40ul of a 1M sucrose (made in a 50ml volumetric flask) was used to which was added 2ul of the sucrose C14 stock. Then 1.2ml of the protoplast suspension was placed into a 1.5ml eppendoff tube and 12ul of the 1M sucrose C14 solution was placed in the cap. The incubation time started when the cap was sealed onto the eppendoff tube. Next, 100ul aliquots of the protoplasts were taken at 10 minute intervals and placed sequentially into a series of the prepared 400ul tubes containing the silicon oil gradients and spun at 7000rpm for 10 second. The tubes were removed at each time interval and cut through the upper silicon oil fraction to capture the formed protoplast pellet and resuspended in 100ul of Milli Q water in a scintillation tube containing 3ml of scintillation fluid. As a standard, 100ul volumes of the 1M sucrose containing the sucrose C14 were taken for counting but not spun through the silicon oil as were the protoplasts without the isotope but these spun through the silicon oil and counted to avoid quenching correction of the protoplast extract.

A graph of time vs. counts, recorded as nmoles of absorbed sucrose was then prepared to confirm the uptake of our sucrose isotope by the competent protoplasts.

### Example 4: Endocytosis studies

### Incubation of formed protoplasts with dye bound liposomes:

An 0.8ml aliquot of protoplast suspension was dispensed through a large orifice pipette tip (ensuring minimal disturbance of the fragile protoplast) into a sterilised eppendoff tube to which was added 0.8ml of the liposome suspension obtained from the gel filtration. Both solutions were of equivalent densities to ensure the stability of the protoplast and gently mixed prior to incubation. The tubes were individually wrapped in foil and embedded on their side in a sheet of Styrofoam which was also wrapped in foil to ensure the tubes would remain in place and then immersed into a water bath a 37°C for the 90 minute incubation.

### Confocal microscope studies identifying liposomal fusion and fluorescent endocytosis:

A 20ul sample of the incubated suspension was applied to a microscope slide with the edges of the cover slip sealed with an acrylic resin to inhibit drying out of the sample. The slides once made were stored in the dark to reduce the risk of a loss in fluorescence of the FITC conjugate and viewed immediately under phase contrast and fluorescence using the confocal microscope.

### Transmission Electron Microscope studies confirming liposomal coalescence and cellular endocytosis:

The technique used for preparing sections of block-mounted cells for image analysis required 0.5ml of the protoplast-liposome suspension to be fixed with 50ul of 1.25% (v/v) glutaraldehyde and 1.0% (w/v) paraformaldehyde in a 0.2M cacodylate buffer at pH 7.2 for 1 hour. The fixed suspension was then mixed with a similar volume of 5.0% agarose and placed at 4°C for 20 minutes to set. Small cubes of the mixture (approximately 2mm³) were cut and fixed for a further 14 hours at 4°C. Samples were then post fixed with 1.0% (w/v) osmium tetroxide, stained *en bloc* with 2.0% (w/v) uranyl acetate, dehydrated through a graded ethanol series and embedded in Epon/Araldite resin.

Ultra thin sections were cut on a Leica ultramicrotome and collected on copper/palladium grids. Sections were stained with 4.0% (w/v) uranyl acetate and Reynolds lead acetate and viewed with a Philips CM 100 Transmission Electron Microscope with images captured on a Gatan Dual Vision digital camera.

### Example 5: Production and use of a liposome comprising 5'-phosphodiesterase

Barley rootlets are macerated in a Waring blender. The resulting solution is then filtered through a cheese cloth and spun at 10,000 rpm at 4°C for 20 minutes to remove the root debris. The supernatant is removed and heated for 1 hour at 60°C. The solution is again spun at 10,000 rpm and 4°C for 20 minutes and the supernatant collected and desalted in a Hitrap 26/10 desalting column on an FPLC. The desalted solution is passed through an anionic exchange using a High Prep 16/10 column and active fractions collected, desalted and then passed through a Mono Q column. The single purified enzyme is then concentrated on a ultrafiltration membrane by centrifugation through an Amicon Ultra 4 membrane with a 30,000 wt cut off at 4°C and 7,500xg for 8 minutes.

Liposomes are then formed by a process of solvent evaporation and freeze drying to produce a thin film of phospholipids in the desired concentration of PC 50%, DOPE 30%, DOTAP 10%, and Ergosterol 10%. The films are hydrated first in Milli Q water and then in the desalted enzyme solution containing an osmotically stabilised buffer of 0.65M sorbitol to ensure the 5'-phosphodiesterase will not become inactive on freezing and thawing. The formed multilamellar vesicles are briefly sonicated for 2 minutes in a bath sonicator to reduce aggregation and continue the formation of large liposomes and then frozen and thawed 3 times to increase the capture potential of the liposomes. The thawed solution of liposomes is then extruded through an Avestin 400nm pore size filter extruder with large unilamellar vesicles containing the enzymes isolated by gel filtration on the FPLC using a Sepharose CL 4B filled 30cm by 1cm column. The enzyme bound liposomes are then concentrated again on the Amicon Ultra 4 centrifuge tube and stored at 4°C until required.

Protoplasts and spheroplasts are prepared as described above. The protoplasts are then concentrated by centrifugation on a Millipore Ultra Free CL 5 µm membrane tube for 50 minutes at ambient temperature and 900 rpm after a 1:4 dilution in the 1.03g/l gradient buffer is undertaken to aid centrifugation. The concentrated protoplasts are then combined 50:50 with the concentrated enzyme bound liposome in an eppendorf tube and incubated in a shaking water bath at 37°C for 90 minutes to effect the transfection of the liposomes and to facilitate the release of the bound enzyme. The mixture of cells is then lysed on a French press and the cell wall material spun down at 10,000 rpm and 4°C for 20 minutes and the supernatant collected and frozen to retard any further reaction of the released enzyme. The 5' ribonucleotides in solution are then measured by RPHPLC against an incubated yeast protoplast suspension without the addition of the enzyme bound liposomes and the values compared.

### Example 6: Results and Discussion

We incorporated phospholipids, PE and PC into the primary matrix of a liposome. The inclusion of a charged amphiphile, DOTAP was to incite initial fusion to the surface proteins found on the yeast plasma membrane and the stabilising agent. Ergosterol was used to facilitate an easier lipid exchange between the yeast cell wall and the liposome. Additional compounds have also been built in to the liposome structure to facilitate a controlled release of the encapsulated material and are based on PE and its derivative DOPE which contain a unique high degree of unsaturated fatty acids.

PE was used with DOPE in the release mechanism for our liposomes, it has a smaller head group compared to PC and is inherently cone shaped and prone to forming hexagonal shaped, inverted micelles in solution particularly at a lower pH. The presence of PE and particularly DOPE with two unsaturated oleic groups provides for a higher membrane curvature within the liposome that leads to a faster facilitated release of the encapsulated material and as such was incorporated to operate as the trigger release compound within the formula.

The trials that were undertaken comprised the following phospholipid combinations:

| | | | | | |
|---|---|---|---|---|---|
| 1. | Phosphatidylcholine | - 50 molar %(125ul) | 7. | Phosphatidylcholine | - 50 molar %(125ul) |
| | DOPE | - 40 molar %(100ul) | | Phosphatidylethanolamine | - 50 molar % (125ul) |
| | DOTAP | - 10 molar % (25ul) | | | |
| 2. | Phosphatidylcholine | - 50 molar %(125ul) | 8. | Phosphatidylcholine | - 50 molar %(125ul) |
| | DOPE | - 30 molar % (75ul) | | Phosphatidylethanolamine | - 40 molar %(100ul) |
| | DOTAP | - 20 molar % (50ul) | | DOTAP | - 10 molar % (25ul) |
| 3. | Phosphatidylcholine | - 40 molar %(100ul) | 9. | Phosphatidylcholine | - 50 molar %(125ul) |
| | DOPE | - 40 molar %(100ul) | | Phosphatidylethanolamine | - 30 molar %(75ul) |
| | DOTAP | - 20 molar % (50ul) | | DOTAP | - 20 molar % (50ul) |
| 4. | Phosphatidylcholine | - 50 molar % (125ul) | 10. | Phosphatidylcholine | - 40 molar %(100ul) |
| | DOPE | - 30 molar % (100ul) | | Phosphatidylethanolamine | - 40 molar % (100ul) |
| | DOTAP | - 10 molar % (25ul) | | DOTAP | - 20 molar % (50ul) |
| | Ergosterol | - 10 molar % (25ul) | | | |
| 5. | Phosphatidylcholine | - 40 molar %(100ul) | 11. | Phosphatidylcholine | - 50 molar %(125ul) |
| | DOPE | - 30 molar %(75ul) | | Phosphatidylethanolamine | - 30 molar % (100ul) |
| | DOTAP | - 20 molar % (50ul) | | DOTAP | - 10 molar % (25ul) |
| | Ergosterol | - 10 molar % (25ul) | | Ergosterol | - 10 molar % (25ul) |
| 6. | Phosphatidylcholine | - 100 molar % (250ul) | 12. | Phosphatidylcholine | - 40 molar %(100ul) |
| | | | | Phosphatidylethanolamine | - 30 molar % (100ul) |
| | | | | DOTAP | - 20 molar % (50ul) |
| | | | | Ergosterol | - 10 molar % (25ul) |

All trials were undertaken in duplicate using a unencapsulated FITC conjugated dextran as a negative control to confirm non-endocytosis transport by an unencapsulated conjugate.

There are three major categories of liposomes, Multilamellar vesicles which contain 2 or more concentric lamellae arranged in an onion skin configuration and can range in size form 1um to <100um, Large Unilamellar Vesicles which have a single bilayer and size distribution in the range of 200um to 1um and Small Unilamellar Vesicles which are less than 200nm in size. For our work, we have chosen the Large Unilamellar Vesicles (LUV) because Multi Lamellar vesicles were to large to cross the yeast plasma membrane and Small Unilamellar Vesicles were too small and unable to trap significant quantities of our FITC Dextran or 5'-phoshpodiesterase.

Large Unilamellar Vesicles can be made by a variety of methods after initial hydration of the lipid film to form MLV. Probe or bath sonication is a time consuming process that produces liposomes of unequal size and capture potential and in the case of probe sonication produces only a small volume of suspension which needs to be cooled in ice water to remove the heat generated from the tip of the probe. This process is not scalable or adaptable to the encapsulation of enzymes as the heat generated and the requirement to remove titanium fragments from the liposome suspension would result in the inactivation the enzymes to be encapsulated.

A dehydration-rehydration procedure can also be adopted and is scalable to 1001ts volumes but is also variable in the capture potential. Reverse Phase solvent evaporation Ethanol Injection, detergent dialysis can also be applied but the drawback of each of these protocols while currently used by many researchers, exposures our enzyme to either organic solvents or detergents which will significantly reduce its activity. The detergent removal procedure particularly requires exhaustive dialysis to remove the surfactants and would be prohibitively expensive for the food industry.

Micro fluidisation has also be used to prepare vesicles as can a French Press, but again enzyme denaturation by shear forces would make these procedures unacceptable.

We used solvent evaporation for the lipids and a gentle freeze thaw and extrusion protocol for the production of LUVs because it allowed for a high capture volume of the fluorescent dye and the 5'-phosphodiesterase and permitted the easier incorporation of a cationic lipid.

The inclusion of a preliminary freeze-thaw step prior to extrusion provides for and increase in the capture volume within the core of the formed LUV as confirmed also by our TEM observations. The principle of freezing and thawing causes a rupture and refusion of the liposome during which time the encapsulated solute equilibrates in the deeper cavities of the liposomal core. The liposomes upon freezing and thawing can also aggregate however it was seen that a brief sonication step prior to extrusion could disassociate the cluster and aid in the formation of LUVs.

In experiments with calcein, up to 6 freeze thaw cycles were used to increase the capture volume however in the case of the FITC conjugated Dextran, only 3 cycles were preformed in an effort to avoid breaking off the fluorescein compound which if free could provide a false positive result as was seen in the endocytosis studies with Calcein.

The size of the formed liposomes was tailored to < 400nm with the action of the extruder reducing the risk of denaturing enzymes or conjugates as well as fouling the membranes. The filter used were a 400mm filter although a 200nm filter was later evaluated to increase the percentage of small LUV for easier migration across the lipid membrane of the cell. The polycarbonate membranes used for the extrusion are produced by a combination laser and chemical etching process, which aims to produce straight-sided pore holes of exact diameter.

It was also found that during our trials that there was a necessity to hydrate and extrude our lipids above the phase transition temperature of the highest temperature lipid to avoid tearing of the polycarbonate filter pads during extrusion.

The extrusion procedure produced LUVs of a uniform, defined size as determined by the Malvern Mastersizer S, and confirmed by Transmission Electron Micrographs. The average size was 0.36 of a micron.

The process of gel filtration was used to fractionate the formed liposomes from the solution containing the unencapsulated dye. The successful separation of the cationic liposomes required an understanding of the principles of separation based on a differential size of the materials and the capability to pack an efficient gel filtration column. Sepharose CL 4B was the packing material used for the separation and is a bead-formed agarose which is derived from agar and cross-linked by reaction with 2,3 dibromopropanol under alkaline conditions. The cross-linking effect of this material as in the case of cross-linked starches provides the agarose gel with a greater thermal and chemical stability over a wide pH range.

Sepharose CL 4B chosen because it is in the middle of the separation range for Sepharose and has a smaller bead size within its bottom range which facilitates a faster movement of larger molecular weight components. It has an optimum separation range between 70 x 10² - 20 x 10² and a bead size in the range of 45 to 165um.

The filters for the column were chosen specifically to be 25um to ensure the smaller size beads within the column would remain and the liposomes would separated and not become lodged on to the top of the column filter restricting the flow and hindering the separation as seen when early columns were used for our trials. The conventional filters were identified to be 2um in size causing fouling and restricted the entry of the large liposomes into the column, prohibiting their isolation.

The separation buffer used in the trials was chosen because it could osmotically support the formed protoplasts when combined with the later liposomes for endocytosis studies and identified during the gradient separations for the formed protoplasts. Separation of liposomes from gel filtration was identified by two eluent peaks on the chromotograph in with the milky suspension containing the liposomes exiting the column first as a function of its molecular size and confirming by TEM images, negatively staining on carbon coated grids with 2.0% (w/v) solutions of sodium phosphotungstate adjusted to pH 7.0

Protoplasts were prepared according to standard techniques. A three-hour incubation was undertaken with post centrifugation to isolate the protoplasts performed at 1000rpm for 15minutes at 4°C. Protoplasts were found primarily in the third layer of the falcon tube with the solution having a density of 1.07g/l. This buffer concentration was then used as the separation solution for all gel filtration experiments. Protoplasts were identified by phase contract and TEM microscopy with remnants of cell wall present and captured. It was observed during the confocal and TEM microscopy that remnants of the cell wall containing the negatively charged proteins formed clusters with the DOTAP liposomes particularly at the 20 molar % concentration.

The addition of a two-component enzyme system using Lyticase and Snail Gut Juice, (*β*-Glucuronidase from Helix pomatia) target the hydrolysis of β-(1-3) glucans and the cysteine bonds within proteins of the cell wall to effectively hydrolyse both carbohydrates and proteins producing the protoplasts. Incubation trials were undertaken at a constant temperature of 37°C and at a pH of 7.2 because we had identified pH not to be a critical parameter and all prior hydrations and purification steps were conducted at this temperature. The only variable that was reviewed was the incubation time, which varied from 30 minutes to 90 minutes. It was decided that 90 minutes would be the most appropriate time to achieve the highest transfected levels of protoplasts.

To ensure the uptake of our liposomes by endocytosis, viable protoplasts had to be determined and the use of a radio labelled isotope of sucrose was adopted with the uptake of this metabolite recorded over a one-hour period. Cells were grown in a sucrose media to facilitate the desired cellular transport mechanism.

Standards were prepared and measured for background scintillation without the added isotope and 100ul of a 1M sucrose standard continuing the isotope was measured to obtain counts for a 1 umole solution of sucrose. The scintillation result showed the 1 umole of sucrose provided approximately 7000counts, therefore 1 count was calculated to be 1 x 10⁻⁶ /7000 or 144 x 10⁻¹² moles. Background counts for the non-isotope sucrose solution was 9 counts. The result from this experiment showed a linear and progressive uptake of the sucrose isotope as identified in the table below highlighting the competent and viable nature of the formed protoplasts.

**Table 1. Time and scintillation counts for the determination of I-Sucrose 14 C uptake by yeast protoplasts**

| Time (mins) | Scintillation count | Scintillation count - background count | I-Sucrose 14 C uptake (nmoles) |
|---|---|---|---|
| 1 | 22 | 13 | 1.87 |
| 10 | 55 | 46 | 6.62 |
| 20 | 102 | 93 | 13.4 |
| 30 | 133 | 124 | 17.9 |
| 40 | 194 | 185 | 26.6 |
| 50 | 258 | 249 | 35.9 |
| 60 | 404 | 395 | 56.9 |

Movement of solutes across the membrane can be undertaken in a number of ways, depending on the size and the charge of the solutes by the process of either diffusion or adsorption. Fusion, inter membrane transfer and exchange of lipids (between liposomes and cell membranes) results in the facilitated uptake of the large molecular compounds by cells with their delivery into early endosomes. The uptake process occurs by the action of either phagocytosis but usually endocytosis and requires solutes to be encapsulation in a micelle with an opposite surface charge to that of the cell.

During endocytosis, an initial coalescence of liposomes with a cell's plasma membrane occurs when the cell's surface proteins or enzymes that are negatively charged bind to a positive charge that has been applied onto the liposome with the further possible addition of stabilising cations, antibodies or Proplylene Glycol to ensure protection and correct orientation of the functionally active enzymes bound to the cells surface.

Once fixed to the surface of the cell, the liposome becomes enveloped by the plasma membrane.

Once brought into the cell, the liposome resides in an early endosome. The action of a reduced pH environment within the cell of between 5.6 and 5.9 combined with an elevated incubation temperature of 37°C encourages the exchange of lipids between the 2 membranes resulting in a weakening of the lipsome and endosomal structure.

At an elevated temperature, the fluidity of the LUV and the alteration in the packing density caused by the ergosterol aided in the facilitated the release of the conjugated dextran into the cell cytoplasm. The main release mechanism as identified in our work was the PE and DOPE components which contained Oleic acid as identified by the fluorescence seen within the cells with the use of formulas 4, 5, 11 and 12.

**Table 2. A summary of the endocytosis trials with the various liposomal formulas containing FITC Dextran**

| All trials were conducted in duplicate with a negative control evaluating the endocytosis of unencapsulated Dextran | | | |
|---|---|---|---|
| 1 | PC | 50 molar % | Some fusion with the protoplast was seen under confocal, endocytosis was not observed |
| | DOPE | 40 molar % | |
| | DOTAP | 10 molar % | |
| 2 | PC | 50 molar % | Additional cell fusion was observed between the two membranes with clumping of cell remains around the cationic liposomes also observed, there was no indication of endocytosis |
| | DOPE | 30 molar % | |
| | DOTAP | 20 molar % | |
| 3 | PC | 40 molar % | Again, a higher degree of fusion was seen with the liposomes found around the outside walls of the yeast protoplasts. A higher degree of clumping was seen, no indication of endocytosis. |
| | DOPE | 40 molar % | |
| | DOTAP | 20 molar % | |
| 4 | PC | 50 molar % | This was the first indication of endocytosed liposomes as identified by fluorescing cells as confirmed by confocal phase contrast and fluorescence overlay and Transmission Electron Microscopy showing liposomes in the cell cytoplasm. |
| | DOPE | 30 molar % | |
| | DOTAP | 10 molar % | |
| | ERGO | 10 molar % | |
| 5 | PC | 40 molar % | There was signs of endocytosis in the confocal images, not as distinct as in trial 4 but contained more fusion between the liposomes and protoplasts. Additional time may enhance the delivery. It appears that Ergosterol and a higher PC content may be an advantage. |
| | DOPE | 30 molar % | |
| | DOTAP | 20 molar % | |
| | ERGO | 10 molar % | |
| 6 | PC | 100 molar % | No fusion or endocytosis seen. |
| 7 | PC | 50 molar % | No fusion or endocytosis seen. |
| | PE | 50 molar % | |
| 8 | PC | 50 molar % | Again, some fusion seen between the cationic liposomes and the cell protoplasts, no endocytosis. |
| | PE | 40 molar % | |
| | DOTAP | 10 molar % | |
| 9 | PC | 50 molar % | Additional clumping seen between the cationic liposomes and the free cell wall material with liposomal fusion observed |
| | PE | 30 molar % | |
| | DOTAP | 20 molar % | |
| 10 | PC | 40 molar % | Fusion was again observed with clumping of cells and cell remnants around the cationic liposomes |
| | PE | 40 molar % | |
| | DOTAP | 20 molar % | |
| 11 | PC | 50 molar % | Some fluorescence was seen inside the cell but was not as pronounced as the liposomes containing DOPE. the release mechanism appears to require DOPE or the addition of Oleic acid into the liposomal matrix with an additional cationic charge to compensate for the negative charge on Oleic Acid. |
| | PE | 30 molar % | |
| | DOTAP | 10 molar % | |
| | ERGO | 10 molar % | |
| 12 | PC | 40 molar % | Enhanced liposomal fusion was seen with the slight appearance of glowing cells. DOTAP appears to contribute to increased fusion with the outer cell membrane of the protoplast but the presence of a higher PC with Ergosterol component appears to enhance the progress of endocytosis. |
| | PE | 30 molar % | |
| | DOTAP | 20 molar % | |
| | ERGO | 10 molar % | |
| 13 | FITC Dextran | | No fusion or endocytosis |

### Confocal and Electron microscopy determinations of cellular fusion and endocytosis

The confocal images were used to identify those liposomes undertaking active endocytosis with a controlled release of the encapsulated fluorescent dye in to the yeast protoplasts. Confirmation of endocytosis was confirmed by Transmission Electron Microscopy, which provided images of protoplast-liposomes fusion and both small and large liposomes bound to the cell wall as well as small liposomes found within an early endosome of a yeast cell cytoplasm.

### Conclusion:

The action of DOTAP to facilitate coalescence between the liposomes and the yeast protoplast appears to be essential in facilitating cellular fusion while the addition of DOPE particularly provides for a greater release of the bound conjugated dextran compared to the liposomes containing phosphatidylethanolamine, however both work to various degrees.

It can be concluded then the cationic liposomes which are formed at a higher pH than the target cell's cytoplasmic pH are capable of fusing and entering a cells cytoplasm.

The action of a trigger release mechanism incorporating phosphatidylethanolamine is important for the release of the components bound within the liposome however PE alone can also still provide a controlled release of the liposomal contents if the temperature and time of incubation is increased.

## Claims

1. A liposome for fusing with a yeast cell, the liposome **characterised in that** 40-50 molar % of the liposome lipid bilayer is phosphatidyl choline (PC), 10-20 molar % of the liposome lipid bilayer is a cationic amphiphile, about 10 molar % of the liposome lipid bilayer is a sterol and about 30 molar % of the liposome lipid bilayer is phosphatidyl ethanolamine (PE) and/or dioleoylphosphatidylethanolamine (DOPE).

2. A liposome according to claim 1 wherein 50 molar % of the liposome lipid bilayer is PC.

3. A liposome according to claim 1 wherein 20 molar % of the liposome lipid bilayer is cationic amphiphile.

4. A liposome according to claim 1 wherein about 30 molar % of the liposome lipid bilayer is PE

5. A liposome according to claim 1 wherein about 30 molar % of the liposome lipid bilayer is DOPE.

6. A liposome according to claim 1 wherein the sterol is ergosterol.

7. A liposome according to claim 1 wherein the sterol is cholesterol.

8. A liposome according to claim 1 wherein the cationic amphiphile is 1,2-dioleoyl-*sn*-glycero-3-trimethylammonium-propane (DOTAP).

9. A liposome according to claim 1 wherein the liposome lipid bilayer further comprises oleic acid.

10. A liposome according to claim 9 wherein about 5 molar % of the liposome lipid bilayer is oleic acid.

11. A liposome according to claim 1 wherein the liposome has a diameter between 100 and 400 nm.

12. A liposome according to claim 1 wherein the liposome lipid bilayers destabilise at a pH of 5.0 to 6.0.

13. A liposome according to claim 1 wherein the liposome further comprises an exogenous molecule, said exogenous molecule being a molecule selected from the group consisting of a protein, enzyme, amino acid, nucleic acid, sugar and mono sodium glutamate.

14. A liposome according to claim 13 wherein the enzyme is a 5' phosphodiesterase.

15. A liposome according to claim 14 wherein the 5' phosphodiesterase is phosphodiesterase 1 (orthophosphoric diester phosphohydrolase, EC 3.1.4.1).

16. A liposome according to claim 13 wherein the enzyme is 5'-adenyl deaminase.

17. A process for producing a yeast cell spheroplast or protoplast comprising an exogenous molecule comprising contacting a yeast cell spheroplast or protoplast with a liposome as defined in any one of claims 13 to 16 comprising the exogenous molecule to permit the spheroplast or protoplast to receive the liposome.

18. A process according to claim 17 wherein the exogenous molecule is an enzyme.

## Patentansprüche

1. Liposom zum Fusionieren mit einer Hefezelle, wobei das Liposom **dadurch gekennzeichnet ist, dass** 40-50 Mol-% der Lipiddoppelschicht des Liposoms Phosphatidylcholin (PC) sind, 10-20 Mol-% der Lipiddoppelschicht des Liposoms ein kationisches Amphiphil sind, etwa 10 Mol-% der Lipiddoppelschicht des Liposoms ein Sterin sind und etwa 30 Mol-% der Lipiddoppelschicht des Liposoms Phosphatidylethanolamin (PE) und/oder Dioleoylphosphatidylethanolamin (DOPE) sind.

2. Liposom gemäß Anspruch 1, wobei 50 Mol-% der Lipiddoppelschicht des Liposoms PC sind.

3. Liposom gemäß Anspruch 1, wobei 20 Mol-% der Lipiddoppelschicht des Liposoms ein kationisches Amphiphil sind.

4. Liposom gemäß Anspruch 1, wobei etwa 30 Mol-% der Lipiddoppelschicht des Liposoms PE sind.

5. Liposom gemäß Anspruch 1, wobei etwa 30 Mol-% der Lipiddoppelschicht des Liposoms DOPE sind.

6. Liposom gemäß Anspruch 1, wobei es sich bei dem Sterin um Ergosterin handelt.

7. Liposom gemäß Anspruch 1, wobei es sich bei dem Sterin um Cholesterin handelt.

8. Liposom gemäß Anspruch 1, wobei es sich bei dem kationischen Amphiphil um 1,2-Dioleoyl-sn-glycero-3-trimethylammoniumpropan (DOTAP) handelt.

9. Liposom gemäß Anspruch 1, wobei die Lipiddoppelschicht des Liposoms weiterhin Ölsäure umfasst.

10. Liposom gemäß Anspruch 9, wobei etwa 5 Mol-% der Lipiddoppelschicht des Liposoms Ölsäure sind.

11. Liposom gemäß Anspruch 1, wobei das Liposom einen Durchmesser zwischen 100 und 400 nm hat.

12. Liposom gemäß Anspruch 1, wobei sich die Lipiddoppelschichten des Liposoms bei einem pH-Wert von 5,0 bis 6,0 destabilisieren.

13. Liposom gemäß Anspruch 1, wobei das Liposom weiterhin ein exogenes Molekül umfasst, wobei das exogene Molekül ein Molekül ist, das aus der Gruppe ausgewählt ist, die aus einem Protein, Enzym, einer Aminosäure, Nucleinsäure, einem Zucker und Mononatriumglutamat besteht.

14. Liposom gemäß Anspruch 13, wobei das Enzym eine 5'-Phosphodiesterase ist.

15. Liposom gemäß Anspruch 14, wobei es sich bei der 5'-Phosphodiesterase um Phosphodiesterase 1 (Orthophosphorsäurediester-Phosphohydrolase, EC 3.1.4.1) handelt.

16. Liposom gemäß Anspruch 13, wobei es sich bei dem Enzym um 5'-Adenyl-Deaminase handelt.

17. Verfahren zur Herstellung eines Hefezellen-Sphäroplasten oder -Protoplasten, der ein exogenes Molekül umfasst, umfassend das In-Kontakt-Bringen eines Hefezellen-Sphäroplasten oder -Protoplasten mit einem Liposom, wie es in einem der Ansprüche 13 bis 16 definiert ist, welches das exogene Molekül umfasst, so dass der Sphäroplast oder Protoplast das Liposom aufnehmen kann.

18. Verfahren gemäß Anspruch 17, wobei das exogene Molekül ein Enzym ist.

## Revendications

1. Liposome pour une fusion avec une cellule de levure, le liposome étant **caractérisé en ce que** 40 à 50 % molaire de la bicouche lipidique du liposome est la phosphatidyl choline (PC), 10 à 20 % molaire de la bicouche lipidique du liposome est un amphiphile cationique, environ 10 % molaire de la bicouche lipidique du liposome est un stérol et environ 30 % molaire de la bicouche lipidique du liposome est la phosphatidyl éthanolamine (PE) et/ou la dioléoylphosphatidyléthanolamine (DOPE).

2. Liposome selon la revendication 1, dans lequel 50 % molaire de la bicouche lipidique du liposome est de la PC.

3. Liposome selon la revendication 1, dans lequel 20 % molaire de la bicouche lipidique du liposome est un amphiphile cationique.

4. Liposome selon la revendication 1, dans lequel environ 30 % molaire de la bicouche lipidique du liposome est de la PE.

5. Liposome selon la revendication 1, dans lequel environ 30 % molaire de la bicouche lipidique du liposome est de la DOPE.

6. Liposome selon la revendication 1, dans lequel le stérol est l'ergostérol.

7. Liposome selon la revendication 1, dans lequel le stérol est le cholestérol.

8. Liposome selon la revendication 1, dans lequel l'amphiphile cationique est le 1,2-dioléoyl-sn-glycéro-3-triméthylammonium-propane (DOTAP).

9. Liposome selon la revendication 1, dans lequel la bicouche lipidique du liposome comprend en outre de l'acide oléique.

10. Liposome selon la revendication 9, dans lequel environ 5 % molaire de la bicouche lipidique du liposome est de l'acide oléique.

11. Liposome selon la revendication 1, dans lequel le liposome a un diamètre compris entre 100 et 400 nm.

12. Liposome selon la revendication 1, dans lequel les bicouches lipidiques du liposome se déstabilisent à un pH de 5,0 à 6,0.

13. Liposome selon la revendication 1, dans lequel le liposome comprend en outre une molécule exogène, ladite molécule exogène étant une molécule choisie dans le groupe consistant en une protéine, une enzyme, un acide aminé, un acide nucléique, du sucre et du glutamate de monosodium.

14. Liposome selon la revendication 13, dans lequel l'enzyme est une 5' phosphodiestérase.

15. Liposome selon la revendication 14, dans lequel la 5' phosphodiestérase est la phosphodiestérase 1 (phosphohydrolase du diester orthophosphorique, EC 3.1.4.1).

16. Liposome selon la revendication 13, dans lequel l'enzyme est la 5'-adényl désaminase.

17. Procédé de production d'un sphéroplaste ou protoplaste de cellule de levure comprenant une molécule exogène comprenant la mise en contact d'un sphéroplaste ou protoplaste de cellule de levure avec un liposome comme défini dans l'une quelconque des revendications 13 à 16 comprenant la molécule exogène pour permettre au sphéroplaste ou protoplaste de recevoir le liposome.

18. Procédé selon la revendication 17, dans lequel la molécule exogène est une enzyme.
